# EUROPEAN PATENT APPLICATION

(11) **EP 3 354 271 A1**
(43) Date of publication of application: **01.08.2018**
(21) Application number: 16857874.8
(22) Date of filing: 10.10.2016
(51) Int. Cl.: A61K 35/32, A61K 35/60, A61K 31/737, A61K 47/10, A61K 9/08, A61P 19/02

(54) **AGENT FOR TREATING ARTHROLOGICAL DISEASES**

(30) Priority: 19.10.2015 RU 2015144704
(71) Applicant: Limited Liability Company "Diamed-Farma", Korolev, Moskovskaya obl. 141069 (RU)
(72) Inventor: SHESTAKOV, Vladislav Nikolaevich, Moscow 117418 (RU); PERSANOVA, Lyudmila Vasil'evna, Moscow 125171 (RU); SAVEL'EVA, Kristina Romanovna, Vladimirskaya obl. 601787 (RU); ANDREEVICHEVA, Tat'yana Yur'evna, Moscow 125499 (RU)
(74) Representative: Kormos, Ágnes
(86) International application number: PCT/RU2016/000681
(87) International publication number: WO 2017/069660

(57) **Abstract**

The invention relates to the medical field, in particular, to medications for treating arthropathies. The medication is based on a Sodium salt of chondroitin sulfate (CS), a mucopolysaccharide produced from animal tissues. The medicinal product influences metabolic processes in connective tissues, including those in cartilages, promoting and normalizing the biosynthesis of glycosaminoglycans. The essence is that the medication is produced from a chondroitin sulfate Na-salt substance having an intrinsic viscosity (η) of 0.01-0.15 m3/kg and free from peptides. The result is that a medication is produced that meets the requirements applicable to medicinal products containing mucopolysaccharides, in particular, chondroitin sulfate, while being substantially free from such side effects as painfulness of injections, occurrence of indurations and hemorrhages in the injection area, and, occasionally, infiltrates.

## Description

The present invention relates to the medical field, in particular, to a composition of an injectable form of a chondroitin sulfate (CS) medicinal product for treating arthropathies and rheumatic diseases based on a Na-salt of chondroitin sulfate, a mucopolysaccharide from animal tissues. The medicinal product, under the name Mucosat assigned to it by the Russian Ministry of Health, may be used to treat degenerative aging-associated arthropathies and spinal disorders, and severe musculoskeletal injuries. The medicinal product influences metabolic processes in connective tissues, including those in cartilages, promoting and normalizing the biosynthesis of glycosaminoglycans.

This medication is based on a Na-salt of chondroitin sulfate (CS), a mucopolysaccharide produced from animal tissues. The medicinal product influences metabolic processes in connective tissues, including those in cartilages, promoting and normalizing the biosynthesis of glycosaminoglycans.

Chondroitin sulfate is a high-molecular-weight heteropolysaccharide, falling into the group of glycosaminoglycans - linear straight chain polymers.

CS molecular structure is responsible for its polyanionic properties and involvement in the water, amino acid, and lipid transport processes in avascular areas of a cartilage. Long chains of the CS, forming part of an extracellular matrix, provide for the key biomechanical properties of the cartilaginous tissue.

Chondroitin sulfate is a structural modulator, not only synthesized by the body, but also, once injected, integrating into the cartilaginous tissue structures, promoting the synthesis and inhibiting the destruction thereof. If timely prescribed and taken on a regular basis, it provides for retarding, stabilization, and prevention against destructive processes in a joint. Chondroitin sulfate's main function in a body is to retain water and nutrients in a cartilage and to provide for molecular motion through a cartilaginous tissue. It is a vital feature, since cartilages do not contain blood to provide for metabolic exchange.

Chondroitin sulfate is also a cartilage protector. Water, retained by it deep in a cartilage, provides good buffering and shock absorption, thereby enhancing the connective tissue strength.

Two groups of chondroprotective medicinal products are currently commercially available.

The first group includes bioactive concentrates of animal tissues:
Alflutop (BIOTEHNOS, Rumania), a medicinal product which is a bioactive concentrate produced from small-size sea fish, and Rumalon (Robapharm, Switzerland), a medicinal product produced from mucopolysaccharides extracted from sternum cartilages and bone marrow of calves. These medicinal products have relatively high therapeutic potency; however, high peptide content in them (up to 8%) causes allergies, occasionally severe, in rather large patient groups, thus preventing further use of these medicinal products. These medicinal products are contraindicated during pregnancy and lactation.

The second group of medicinal products includes chondroitin sulfate in the form of a pharmaceutical substance.

Known is a medicinal product Mucosat, a solution for injection, comprising a mixture of chondroitin 4-sulfate (A) and chondroitin 6-sulfate (C) (8-12%), 0.8-1.2% of benzyl alcohol as a preservative agent, and pyrogen-free water as the balance, the medicinal product solution pH being 6.5-7.5.

Chondroitin sulfate pharmaceutical substance manufacturers do not currently include the chondroitin 4-sulfate (A) to chondroitin 6-sulfate (C) ratio or molecular weight into their quality control criteria. Molecular weight values of the CS substance from which an injectable medicinal product is produced determine the product's viscosity and thus, the painfulness of injections, occurrence of indurations and hemorrhages in the injection area, and, occasionally, infiltrates.

An object of the present invention is to identify a range of chondroitin sulfate sodium salt substance's intrinsic viscosity values that enable producing a medicinal product, by the use of which a technical result may be achieved, consisting in the elimination of such effects as painfulness of injections, occurrence of indurations and hemorrhages in the injection area, and, occasionally, infiltrates.

A set of experiments has been carried out as part of the claimed medicinal product development to review viscous properties of chondroitin sulfate substances produced by various manufacturers.

As required by the European and British Pharmacopoeias, intrinsic viscosity (η) for chondroitin sulfate Na-salt substances shall be within the range from 0.01 to 0.15 m3/kg.

The experiment has demonstrated that the substances with an intrinsic viscosity (η) of 0.01 to 0.15 m3/kg may be used to produce an injectable form of a chondroitin sulfate Na-salt based medicinal product of appropriate quality.

A medicinal product is provided, comprising: 4-12% of chondroitin sulfate Na-salt (preferably, 10 % wt) with an intrinsic viscosity of 0.01 to 0.15 m3/kg (preferably, 0.029 to 0.04 m3/kg), benzyl alcohol as a preservative agent (0.8-1.2%), and pyrogen-free water as the balance, the medicinal product solution pH being 5.5-7.5 (pH is adjusted with a 1 N sodium hydroxide solution).

The medicinal product may further comprise 0.09 - 0.11 % wt of sodium bisulfite as an additional preservative agent and anti-oxidant.

*Example 1.* Solution A preparation. 100.0 g of CS (expressed as the basic substance), η=0.01 m3/kg (manufactured by Syntex (Argentina IHS: 42-12641-02) is dissolved in 500 ml of water for injections.

Solution B preparation. 350 ml of water for injections is added to 10.0 g of benzyl alcohol and stirred until benzyl alcohol is completely dissolved.

Preparation of the medicinal product solution. With constant stirring, the Solution A is added with 350 ml of the Solution B, 0.1 M NaOH solution to adjust pH to 7.0, and water for injections to make up to a volume of 1 1. A solution is produced with the chondroitin sulfate content of 10 % wt and benzyl alcohol content of 1 % wt. The solution is sterile filtered and dispensed into 1.0 or 2.0 ml ampoules. The ampoules are then vacuum-sealed. In this way, an injectable medicinal product for intramuscular injection is produced with a chondroitin sulfate content of 10 % wt and benzyl alcohol content of 1 % wt.

*Example* 2. Solution A preparation. 100.0 g of CS (expressed as the basic substance), η =0.029 m3/kg (manufactured by Bioiberica S.A., Spain. IHS: 42-12952-03) is dissolved in 500 ml of water for injections,.

Solution B preparation. 350 ml of water for injections is added to 8.0 g of benzyl alcohol and stirred until benzyl alcohol is completely dissolved.

Preparation of the medicinal product solution. With constant stirring, the Solution A is added with 350 ml of the Solution B, 0.1 M NaOH solution to adjust pH to 7.0, and water for injections to make up to a volume of 1 1. A solution is produced with the chondroitin sulfate content of 10 % wt and benzyl alcohol content of 0.8% wt. The solution is sterile filtered and dispensed into 1.0 or 2.0 ml ampoules. The ampoules are then vacuum-sealed. In this way, an injectable medicinal product for intramuscular injection is produced with a chondroitin sulfate content of 10 % wt and benzyl alcohol content of 0.8 % wt.

*Example 3.* Solution A preparation. 40.0 g of CS (expressed as the basic substance), η =0.040 m3/kg (manufactured by Bioiberica S.A., Spain. IHS: 42-12952-03) is dissolved in 500 ml of water for injections.

Solution B preparation. 350 ml of water for injections is added to 12.0 g of benzyl alcohol and stirred until benzyl alcohol is completely dissolved.

Preparation of the medicinal product solution. With constant stirring, the Solution A is added with 350 ml of the Solution B, 0.1 M NaOH solution to adjust pH to 6.5, and water for injections to make up to a volume of 1 1. A solution is produced with the chondroitin sulfate content of 4 % wt and benzyl alcohol content of 1.2% wt. The solution is sterile filtered and dispensed into 1.0 or 2.0 ml ampoules. The ampoules are then vacuum-sealed. In this way, an injectable medicinal product for intramuscular injection is produced with a chondroitin sulfate content of 4 % wt and benzyl alcohol content of 1.2 % wt.

*Example 4.* Solution A preparation. 120.0 g of CS (expressed as the basic substance), η= 0.15 m3/kg (manufactured by Sintez AKO, Russia, IHS: MPM 42-0054-5605-04) and 1.0 g of sodium bisulfite are dissolved in 500 ml of water for injections.

Solution B preparation. 350 ml of water for injections is added to 10.0 g of benzyl alcohol and stirred until benzyl alcohol is completely dissolved.

Preparation of the medicinal product solution. With constant stirring, the Solution A is added with 350 ml of the Solution B, 0.1 M NaOH solution to adjust pH to 6.5, and water for injections to make up to a volume of 1 1. A solution is produced with the chondroitin sulfate content of 10 % wt, benzyl alcohol content of 1 % wt, and sodium bisulfite content of 0.1 % wt. The solution is sterile filtered and dispensed into 1.0 or 2.0 ml ampoules. The ampoules are then vacuum-sealed. In this way, an injectable medicinal product for intramuscular injection is produced with a chondroitin sulfate content of 12 % wt and benzyl alcohol content of 1 % wt.

Review of the medicinal product's specific effect on modelled pathological processes in a cartilaginous tissue has shown that the present medicinal product's properties are similar to those of the mucopolysaccharide-based drugs at the same therapeutic doses.

Information sources:
1. RF Patent 22021812 dd. 30.10.1994;
2. Master Formula for the Mucosat Medicinal product Manufacture PR 42-00482111377-09, FGA State Institute for Blood Substitutes and Medicinal Products. - M., 2009.

## Claims

1. A medication for the treatment of arthropathies, comprising: a chondroitin sulfate Na-salt, water, and a preservative agent, wherein the chondroitin sulfate Na-salt with an intrinsic viscosity (η) of 0.01-0.15 m3/kg and benzyl alcohol as a preservative agent are employed at the following mixing ratio in % wt:
Chondroitin sulfate: 4 - 12;
Benzyl alcohol: 0.8 - 1.2;
Water: the balance

2. The medication of claim 1, wherein the chondroitin sulfate content is, preferably, 10% wt.

3. The medication of claim 1, wherein intrinsic viscosity (η) of the chondroitin sulfate substance is, preferably, of 0.029 to 0.04 m3/kg.

4. The medication according to each of claims 1 - 3, wherein sodium bisulfite in the amount of 0.09 to 0.11 % wt is further used as a stabilizer.
